# EUROPEAN PATENT APPLICATION

(11) **EP 4 397 234 A1**
(43) Date of publication of application: **10.07.2024**
(21) Application number: 23150601.5
(22) Date of filing: 06.01.2023
(51) Int. Cl.: A61B 5/0536, A61B 5/257, A61B 5/259, A61B 5/00

(54) **ELECTRICAL IMPEDANCE IMAGING ASSEMBLY**

(71) Applicant: SenTec AG, 4106 Therwil (CH)
(72) Inventor: KRAMMER, Peter, 7000 Chur (CH)
(74) Representative: Hepp Wenger Ryffel AG

(57) **Abstract**

The present invention is related to an electrical impedance imaging assembly (10), an electrical impedance imaging device, an electrical impedance imaging set and a method for manufacturing an electrical impedance imaging assembly. The electrical impedance imaging assembly (10) comprises at least one electrode patch (11, 21) to be externally applied to a patient comprising a plurality of electrodes (12, 22) for measuring impedances of a measurement target placed between the plurality of electrodes (12, 22), and a first interface (13) for transmitting electrical signals between the electrode patch (11, 21) and an external device (100) which computes characteristics of the measurement target based on electrical signals of the electrodes (12, 22), in particular a three-dimensional tomographic image (101) of the measurement target. At least a first electrode patch (11) comprises at least 12 electrodes (12), preferably 16-32 electrodes, the electrodes (12) being arranged on a substrate (14) of the first electrode patch in a two-dimensional pattern, preferably in a matrix shape. The first electrode patch (11) comprises an adhesive (15) for fixing the first electrode patch (11) on the skin of the patient, wherein preferably the ratio between the area of the surface (16) of the adhesive for contacting the skin and the area of the total surface (17) of the electrodes (12) facing the skin is 0.5-2.

## Description

The present invention is related to an electrical impedance imaging assembly, an electrical impedance imaging device, an electrical impedance imaging set and a method for manufacturing an electrical impedance imaging assembly.

Electrical impedance tomography (EIT) is a non-invasive imaging technique based on the application of current and measurement of voltage through electrodes attached to the body of a patient. EIT allows visualizing and monitoring a cross-section of parts of the human body, by capturing its electrical potential. The electrical impedance distribution measured in the body parts is transformed, with the help of an image reconstruction algorithm, in a two-dimensional or three-dimensional image. The image or sequences of images show differences in the electrical properties of various body tissues, bones, skin, body fluids and organs, particularly the lungs, which are useful for monitoring the patient's condition. Capturing the electrical potential of a plurality of electrodes placed on the surface of a patient's chest allows continuous monitoring of lung ventilation and perfusion.

Electrodes used for electro impedance measurements and in particular for electro impedance tomography (EIT) may be placed individually on the thoracic surface to form a measurement plane or a measurement volume. Such individual electrodes are cumbersome to handle and are bound to be dislocated with patient movement. In order to overcome the above problem, belts or belt-like structures have been designed on which electrodes are mounted. The conventional belt is placed around the chest thereby forming a transverse EIT plane for imaging a full plane through the lungs.

To avoid the electrodes from moving on the body during data acquisition, the belt has to be tightly placed on the desired surface. A radial force component may act as a pressing force on the electrodes and also on the test subject's body. To a certain extent such forces may impede normal chest movement and extension during breathing.

Furthermore, the placement of a belt around a patient's torso and the fixation may be inconvenient for the patient and may take some time. This could be disadvantageous if a quick result is needed.

A belt suitable for patients of different sizes with electrodes which have to be positioned very precisely needs an adjustable application mechanism, which may be cost intensive.

It is known to use an adhesive for fixing electrodes to a patient. EP3291731A1 shows a device for measuring conductivity changes by bioelectrical impedance or electrical impedance tomography technology for non-invasive detection of urine flow from the bladder into the kidney. Electrodes may be arranged on a belt which may incorporate an adhesive to prevent movement.

EP3434177A1 discloses a layered body which for example can be used as a self-adhesive electrode patch for electro-physical measurements, such as EIT.

US7206630B1 discloses an electrode patch for measuring the physiological condition of a subject, and more particularly to an electrode patch for ECG monitoring. Electrode patch may be attached to the subject by adhesive on the lower surface of the base or by adhesive on the electrodes on the lower surface of the base.

However, adhesively fixed electrodes may be difficult to be removed after measurement and the adhesive may have a negative impact on the patient's skin.

It is an object of the present invention to overcome the drawbacks of the prior art and in particular to provide an assembly, a device and a kit which allow to provide a meaningful measurement in a simple and reliable way.

According to the invention these and other objects are solved with an assembly, a device and a kit according to the independent claims.

An electrical impedance imaging assembly comprises at least one electrode patch to be externally applied to a patient. The electrode patch comprises a plurality of electrodes arranged for measuring impedances of a measurement target placed in an area between the plurality of electrodes and/or surrounded by the plurality of electrodes and/or below the plurality of electrodes.

The electrical impedance imaging assembly further comprises an first interface for transmitting electrical signals between the electrode patch and an external device which computes characteristics of the measurement target, for example the lungs of a patient from electrical signals of the electrodes.

The interface may be wire based or wireless. The interface may be configured to sense and/or transmit signals of the electrodes. These signals may be representative for impedance values, for ECG values or any other type of electrical signals haven a physiological meaning.

The interface may comprise a plug or a socket for detachably connecting a respective counterpart connected or connectable to the external device.

Alternatively, the interface may be connectable to the respective counterpart by a wireless connection.

At least a first electrode patch comprises at least 12 electrodes, preferably 16-32 electrodes.

The electrode should be as thin as possible to avoid pressure points. The form of the electrode is ideally round.

As the contact impedance depends on the size of the electrode, the electrode preferably has an area that is smaller than 1cm² to provide a reasonable contact impedance.

The electrode material should provide for a good conductivity, a low DC-voltage offset and a sufficient stability with respect to oxidation during use. Materials which may be used for the electrode comprise Ag/AgCl, carbon, gold, palladium/gold.

The electrodes are arranged in or on a substrate of the first electrode patch in a two-dimensional pattern, for example in a matrix shape. Preferably, the electrodes are arranged such that not all electrodes are positioned on one straight or curved virtual line or on two crossing straight or curved virtual lines.

As the first electrode patch provides a two-dimensional pattern of electrodes, the measurement allows for obtaining information about a distribution of various body tissues over the distance from the first electrode patch.

The first electrode patch may be applied to the front or to the back of a patient.

The first electrode patch comprises an adhesive for fixing the first electrode patch on the skin of the patient, wherein the ratio between the area of the surface of the adhesive for contacting the skin and the area of the total surface of the electrodes facing the skin preferably is 0.5-2.

Adhesive fixation is very quick as no application and/or closing device is needed. The electrode patch may be only placed to the desired place and sticks there. On the other hand, as the adhesive area surface has a small area, the electrode patch may be removed easily after measurement and the adhesive does not affect the skin of the patient unduly.

The adhesive may be removable, such that a new adhesive can be applied to the substrate and/or to the electrodes. The electrode patch may be cleaned after use and may be used again after applying a new adhesive. Alternatively, the electrical impedance imaging assembly may be a single use device.

For an optimal resolution the distance of the electrodes may be selected as large as possible. For a given surface of the substrate, the electrodes should be distributed to a maximum extent.

The electrodes may be placed over the whole surface of the substrate of the electrode patch, also close to the boundary. A boundary region at the periphery of the substrate only has to be kept free from an electrode, if this region is used for applying an adhesive is. Thus, for a given number of electrodes and for a given distance of electrodes, the substrate can have a minimal extent.

The first electrode patch may be designed to cover only a part of the surface of the patient, especially on the front, such that access for other medical sensors or procedures is provided.

Preferably, the electrical impedance imaging assembly surrounds only a part of the thorax, in particular less than 180°.

The first electrode patch may be designed to be fixed only on the ventral surface or on the dorsal surface of the thorax. As only a part of the body circumference is covered by the electrodes, the resolution of the electrical impedance image may drop. However, the electrical impedance imaging assembly has a much smaller extent than usual electrical impedance imaging assemblies, wherein electrodes are arranged on a belt. Hence, less material is necessary, and the electrical impedance imaging assembly can be produced at lower costs.

The electrode patch does not need any further fixation means, such as a textile belt. The electrical impedance imaging assembly can therefore be manufactured without great effort which allows a cost-effective production.

The adhesive may be applied to the electrode patch and may be covered by a strippable protection sheet.

The first electrode patch may be applied to the front or to the back of a patient.

As the electrodes of first electrode patch are not distributed completely around the patient's body, a measurement solely on the basis of the electrodes of the first electrode patch does not provide a complete tomographic image. However, a measurement solely on the basis of the electrodes of the first electrode patch may provide a meaningful measure of the patient's condition, for example may provide monitoring at least a part of the lung volume. This measure may be monitored over time and hence a control of a temporal variation of a patient's condition may be provided.

A change of the condition may be monitored during treatment of a patient, for example during ventilation and/or during an alveolar treatment manoeuvre for finding optimal ventilation parameter.

As the first electrode patch may be applied very quickly, a meaningful measure of the patient's condition may be obtained almost immediately when needed, which could be helpful in an emergency situation.

The adhesive may be arranged on or around each electrode of the first electrode patch, which provides for a reliable and at the same time gentle fixation of the electrodes, without the need of too much adhesive material. The adhesive is mainly provided to fix the electrodes and not the electrode patch as a whole.

Additionally, adhesive may be arranged close to an interface and/or on or around a sensor attached to the electrode patch.

Preferably the adhesive is biologically compatible to the subject. More preferably, a pressure sensitive adhesive is used.

Preferably, a removable adhesive is used, which can easily be removed from the patient's skin.

The adhesive may be conductive, to improve the contact between the electrodes and the skin of the patient.

The adhesives used include but are not limited to for example natural rubber, butyl, styrene block copolymer, SBR, acrylics, and silicone based adhesives.

The electrode patch may comprise a visual marking to ensure a proper positioning with respect to the patient. The visual marking may indicate a position or direction which has to be oriented with respect to the patient's spine direction, the patient's nipples or other anatomical landmarks or which has to be placed in a predetermined distance to an anatomical landmark.

The electrode patch may comprise an electronic unit, for example integrated in the interface. The electronic unit may comprise a storage unit with information about the number of uses of the of the electrical impedance imaging assembly, about a type numbering of the electrode patch, about the intended target group of the electrode patch, and/or other parameter, such as geometry and number of electrodes. The genuity of the product may be approved and/or too many uses may be prevented. An external device of an electrical impedance imaging device may read out the information, may or may not allow operation and/or may adapt the further analysis of the electrode signals depending on the information.

The electrode patch may comprise a gravity sensor. Information about the orientation of the electrode patch when applied to the patient may also be transmitted via the interface and may be used by an external device for depicting the impedance image.

The electrodes of the first electrode patch may be electrically connectable, preferably via the external device, such that any two electrodes can be used for measuring the impedance there between. Hence, the electrodes are freely addressable.

The first interface may electrically be connected to all of the electrodes, may be connected to the electrodes of a further electrode patch and/or may be connected to a further interface on the electrode patch and/or to a sensor arranged on the electrode patch.

Thus, all electric components of the electrical impedance imaging assembly may be addressed by the external device and the external device may receive signals from all electric components.

The maximal distance between two electrodes on the first electrode patch which are electrically connectable for measuring impedance between them, may be 10cm to 20cm, preferably 14cm to 16cm.

For four electrodes being placed in a row on a patch with a width of 45cm, the maximal electrode distance preferably is 15cm.

The maximal distance corresponds to the dimension of the first electrode patch. The first electrode patch should be placed without any problem to the back of a normally proportioned adult.

Electrode patches with electrodes of a smaller maximal distance, can be applied to children or neonates.

The minimal distance between two electrodes on the first electrode patch which are electrically connectable for measuring impedance between them, may be 0.5-2cm, preferably 0.7-1.2cm.

A small patch for neonates may have a width of 8cm to 12cm, preferably 10cm, and may comprise 16 electrodes. For such a patch the electrodes preferably have a minimal distance of 0.3cm-0.7cm, preferably 0.5cm.

Typical dimensions of an electrode patch for different patient groups are summarized in the following table.

| Use Case | Patch Area | Total Length of Patch | Number of Electrodes | Minimal Distance between Electrodes |
|---|---|---|---|---|
| Adult Large | 1100- 1600cm² | 40-60 cm | 16 - 32 | 2.5-3.5 cm |
| Adult Middle | 600-1100 cm² | 30-40 cm | 16 - 32 | 2-2.5 cm |
| Adult Small | 350-600 cm² | 20-30 cm | 16 - 32 | 1.5-2 cm |
| Child | 150-350 cm² | 10-20 cm | 16 - 32 | 0.75-1.5 cm |
| Neonate | 30-150 cm² | 5-10 cm | 16 - 32 | 0.3-0.75 cm |

The larger the distance between two electrodes which are used for measuring impedance between them, the larger the penetration depth of the measurement. Electrodes can therefore be arranged in a pattern with mutual distances as large as possible.

The electrical impedance imaging assembly may comprise at least one second electrode patch, comprising at least one electrode. The second electrode patch is electrically connected to the first electrode patch.

With respect to the first patch the second electrode patch may be placed on the opposite side of the patient. The second electrode patch may be externally applied to the sternum of the patient.

The second patch provides at least one additional electrode for generating at least a rough tomographic image. The number of electrodes of the second electrode patch may be smaller than the number of electrodes of the first electrode patch. The second electrode patch may comprise 1-4 electrodes. The area of the surface of the substrate of the second electrode patch may be smaller than the area of the surface of the substrate of the first electrode patch.

The at least first electrode patch and the at least second electrode patch are connected by a flexible connection having no electrode. The flexible connection may have a length of at least 200-400mm. At least one wire may be arranged in the flexible connection to provide electrical connectivity. Alternatively, a wireless connection can be provided between the second electrode patch and the first electrode patch or between the second electrode patch and the external device.

The flexible connection may be formed by a plastic band and/or may be made from the same material as the substrate of the electrode patches.

The first and the second electrode patch may be formed as one piece, wherein both parts may be applied independently from each other. The electrical impedance imaging assembly with a first and a second electrode patch can still be applied to a patient without the need to turn the patient.

The second electrode patch may be fixed to the patient in the same way as the first electrode patch.

The second electrode patch may comprise an adhesive for fixing the electrode patch to the skin of the patient, wherein the adhesive preferably is arranged on each or around each of the at least one electrode. The ratio between the area of the surface of the adhesive for contacting the skin and the area of the total surface of the electrodes facing the skin may be 0.5-2.

At least one electrode patch may comprise at least one connection area. In the connection area there may be disposed at least one second interface for detachably connecting at least one sensor.

The second interface may be designed to establish a wire based and/or wireless connection.

The sensor may be a temperature, pressure, sound and/or positioning sensor, which may be placed in the connection area.

The second interface may comprise a plug or a socket for detachably connecting a respective counterpart connected or connectable to the sensor.

At least one sensor, preferably being a temperature, pressure, sound and/or positioning sensor may be arranged on the electrode patch.

The first interface may be designed for also transmitting electrical signals between the sensor and the external device.

The electrode patch may comprise an electrode layer provided with the plurality of electrodes.

The electrode patch may further comprise a wire layer, preferably stacked on a top of the electrode layer and provided with a wire electrically connected to the electrodes and/or a circuit layer, preferably stacked on a top of the wire layer and provided with an electrode connection unit electrically connected to the wire.

Additionally, the electrode patch may comprise a padding layer to avoid pressure points.

Electrodes and circuits, as well as markings may be printed on a substrate. The printing ensures a homogenous and thin structure of the electrode patch.

The electrode patch may comprise a substrate, formed of any one or more of a paper product, a natural fiber, a synthetic fiber, non-woven cloth, polymer, plastic, pulp, paper and silicon rubber.

The substrate may comprise a certain elasticity, at least in one direction to provide some comfort for use. A PU-based substrate may be stretchable in two directions along the surface of the substrate.

The stretchability may be limited to 20% of initial length.

Reinforcements may be applied to the substrate, in particular between the electrodes to keep the distances fixed or close to the interface to stiffen the holding region for a plug or connector.

Preferably the substrate is soft in a direction perpendicular to the surface of the substrate, especially on the back in the area of the electrodes, which is directed towards the patient. A "cushion effect" shall be achieved, such that there are no pressure points on the patient's skin.

Preferably, the substrate is air permeable.

The electrode patch may comprise a cover covering the substrate, which is formed of a flexible material or plastic, preferably being equipped with an imprint which facilitates the positioning of the electrode patch on the patient.

The electrodes may be arranged within recesses in the substrate, in particular when a pre-applied gel is provided on the electrodes.

Alternatively, the electrodes may be arranged on the substrate.

The electrical impedance imaging assembly may comprise wires, circuits and/or electrodes which are formed by printing an ink containing a conductive material.

Alternatively, wires, circuits and/or electrodes can be made of adhering cloth, non-woven cloth, paper or a flexible substrate having conductivity, infiltrating a conductive organic matter to the substrate, configuring a metallic sticker or adhering a small metal plate.

According to the invention an electrical impedance imaging device comprises at least one electrical impedance imaging assembly as described above and an external device for receiving an electrical signal measured through the electrical impedance imaging assembly and for computing characteristics of the measurement target based on the electrical signals of the electrodes, in particular a three-dimensional tomographic image of the measurement target. Preferably, the electrical impedance imaging device comprises a display for displaying characteristic data and/or a tomographic image. The external device may have such a display.

In particular, the external device provides an input signal between different or all possible pairs of electrodes. While the input signal is applied to one of the pairs of electrodes, the currents or voltages between the remaining electrodes may be measured. The measured electrical voltages of the body section may be reconstructed into electrical properties or changes of electrical properties by a reconstruction algorithm known as such for electrical impedance tomography. By a data processor of the external device a representation of the distribution impedance values over a volume area of the patient around which the electrodes are placed may be obtained. Thus, preferably the external device calculates a three-dimensional tomographic image of the measurement target.

The best results can be achieved, when multiple injections are combined for pairs of electrodes with different distances.

The external device may also be designed for receiving data of at least one further sensor, such as a temperature, pressure, sound and/or positioning sensor, preferably arranged on the electrode patch.

The electrical impedance imaging device may comprise a control unit disposed for choosing any two electrodes of the electrical impedance tomography assembly for measuring impedances.

A measurement may be performed with a suitable cluster of electrodes, depending on where exactly the electrode patch is positioned on the patient and depending on which and how may electrodes have a sufficient contact to the patent's skin.

The electrical impedance imaging device may comprise a control unit which is designed for receiving an ECG signal from selected electrodes of the electrical impedance tomography assembly. The same electrodes may be used for impedance and for electrocardiography measurement. Alternatively, some of the electrodes may only be used for ECG measurement.

The electrical impedance imaging device may comprise at least one additional electrode or contact, e.g. for connection to the ground or as a guard electrode. The additional electrode may be placed on the patient next to the first and/or second electrode patch.

According to the invention an electrical impedance imaging set comprises at least a first electrical impedance imaging assembly as described above, and a second electrical impedance imaging assembly as described above. The electrical impedance imaging set further comprises an external device for receiving an electrical signal measured through one of the electrical impedance imaging assemblies, for computing characteristics of the measurement target based on the electrical signals, in particular a three-dimensional tomographic image of the measurement target. The external device may comprise a display for displaying a tomographic image.

The electrodes of the first electrical impedance imaging assembly are arranged in first pattern and the electrodes of the second electrical impedance imaging assembly are arranged in second pattern, wherein the first pattern is different from the second pattern.

The first electrical impedance imaging may comprise an electrode patch with a first number of electrodes being arranged close together. The second electrical impedance imaging may comprise an electrode patch with a second number of electrodes being arranged in a greater distance.

A user may hence decide between an electrode patch with a greater sensitivity or with a greater penetration depth.

According to the invention an electrical impedance imaging assembly, preferably as described above, is manufactured by proving a substrate, attaching at least twelve electrodes, preferably sixteen to thirty-two electrodes, in a two-dimensional pattern, preferably in a matrix shape to the substrate. An adhesive is attached to or around each electrode, wherein the ratio between the area of the surface of the adhesive for contacting the skin and the area of the total surface of the electrodes facing the skin is 0.5-2.

According to the invention a method of using an electrical impedance imaging assembly, preferably as described above, comprises the following steps.

At least one electrode patch being part of the electrical impedance imaging assembly is placed to thorax of a patient, such that only a part of the thorax is surrounded by the electrical impedance imaging assembly, in particular less than 180°.

The electrode patch has at least 12 electrodes, preferably 16-32 electrodes, for measuring impedances of a measurement target placed between the plurality of electrodes. The electrodes are arranged on a substrate of the first electrode patch in a two-dimensional pattern, preferably in a matrix shape.

The electrical impedance imaging assembly further comprise a first interface for transmitting electrical signals between the electrode patch and an external device which computes characteristics of the measurement target based on electrical signals of the electrodes, in particular a three-dimensional tomographic image of the measurement target.

In a further step the electrode patch is fixed to the skin of the patient by an adhesive.

The adhesive is preferably applied to the first electrode patch, such that the ratio between the area of the surface of the adhesive for contacting the skin and the area of the total surface of the electrodes facing the skin is 0.5-2.

In a preceding step an electrical impedance imaging assembly with an appropriate electrode patch, which is suitable for the patient to be examined, may be selected from a set of electrical impedance imaging assemblies as described above and/or from a set of electrical impedance imaging assemblies with different sizes.

The electrical impedance imaging assembly may be connected to an external device to establish an electrical impedance imaging device, in particular as described above.

The invention is described with reference to preferred embodiments and the drawings, which show:
- Fig. 1: a schematic presentation of first example of an electrical impedance imaging assembly;
- Fig. 2: a schematic presentation of second example of an electrical impedance imaging assembly;
- Fig. 3: a schematic presentation of an electrode in top view;
- Fig. 4: a schematic presentation of an electrical impedance imaging device.

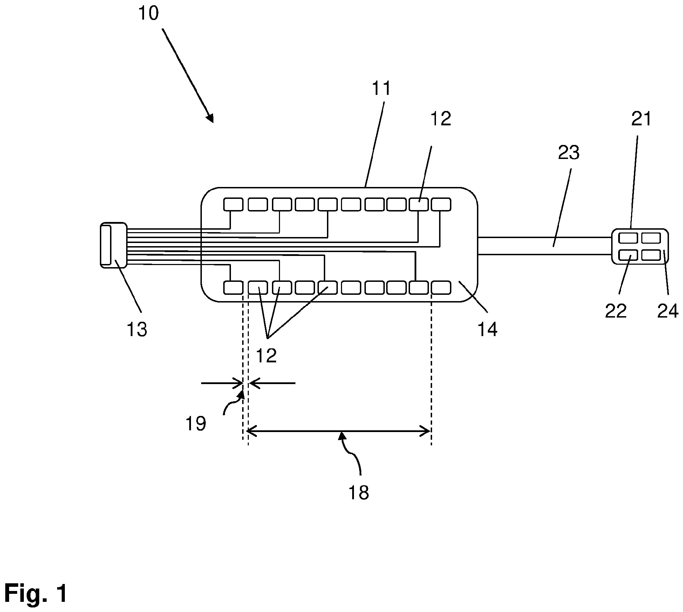

Figure 1 shows a schematic presentation of first example of an electrical impedance imaging assembly 10.

The electrical impedance imaging assembly 10 comprises a first electrode patch 11 and a second electrode patch 21.

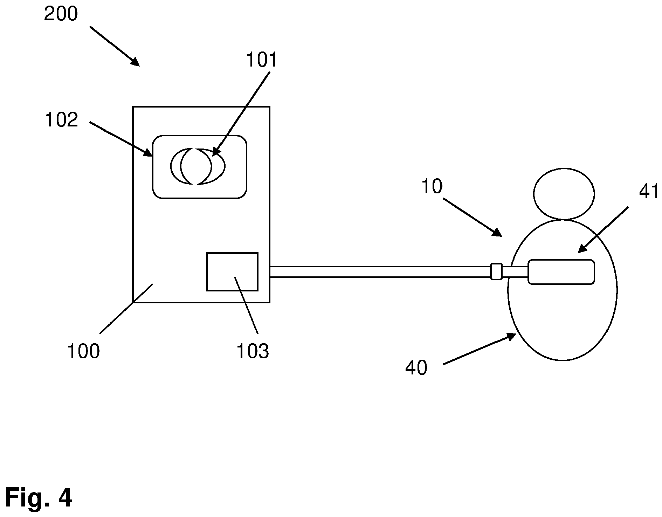

A plurality of electrodes 12 are arranged on a substrate 14 of the first electrode patch 11. Impedances of a measurement target placed between the plurality of electrodes may be measured when the electrode patch 11 is applied to a patient 40 (see figure 4), for example to the back of a patient 40. The electrodes 12 are arranged on the substrate 14 of the first electrode patch 11 in a matrix shape.

The electrical impedance imaging assembly 10 comprises a
a first interface 13 for transmitting electrical signals between the electrode patch 11 and an external device 100 (see figure 4) .

The maximal distance 18 between two electrodes 12 on the first electrode patch 11 which are electrically connectable for measuring an impedance between them depends on the number and kind of electrodes on the patch and on the patient (see table above). This distance allows for a penetration depth of the measurement of about the same lengths as the distance between the electrodes.

The minimal distance 19 between two electrodes 12 on the first electrode patch 11 which are electrically connectable for measuring an impedance between them, is 0.3cm to 2.5cm and depends on the patient (see table above).

The second electrode patch 21 may be externally applied to the sternum of the patient P. The second electrode patch 21 comprises a number of electrodes 22 arranged on a substrate 24.

The number of electrodes 22 of the second electrode patch 21 is smaller than the number of electrodes 12 of the first electrode patch 11.

The second electrode patch 21 is electrically connected to the first electrode patch 11 by flexible connection 23 having no electrode.

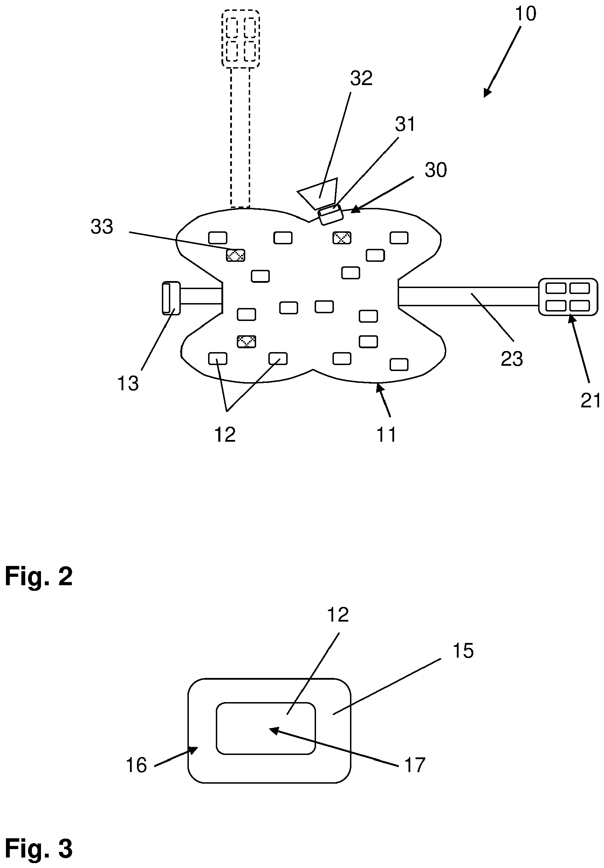

Figure 2 shows a schematic presentation of second example of an electrical impedance imaging assembly 10.

Whereas in the first example the electrodes 12 are arranged in rows (see figure 1), in the second example the electrodes 12 are arranged in a two-dimensional pattern, which covers the contours of the lungs when applied to the back of a patient.

A second electrode patch 21 may be connected to the first electrode patch 11 either such that the flexible connection 23 can be passed around the patient's side to the sternum or the flexible connection 23 (shown in dashed lines) can be passed over the shoulder of the patient to place the second electrode patch 21 on the front of the patient.

The first electrode patch 11 comprises a connection area 30 with a second interface 31.

A sensor 32 is removably connected to the second interface 31.

The second interface 31 is electrically connected to the first interface 13.

Some of the electrodes 33 may be selected to be used for an electrocardiography measurement.

Figure 3 shows a schematic presentation of an electrode in top view.

The first electrode patch 11 (see figure 1 or 2) comprises an adhesive 15 for fixing the first electrode patch 11 on the skin of the patient 40. The adhesive 15 may be arranged around each electrode 12. The ratio between the area of the surface 16 of the adhesive for contacting the skin and the area of the total surface 17 of the electrodes 12 facing the skin is 0.5-2.

Figure 4 shows a schematic presentation of an electrical impedance imaging device 200 comprising an external device 100 and an electrical impedance imaging assembly 10 which is applied to a patient 40.

The external device 100 comprises a control unit 103 which is designed for communication with the electrical impedance imaging assembly 10 and for reconstructing an image 101 of the measurement target 41, typically including at least parts of the lungs of the patient 40.

The external device 100 comprises a display 102 for showing the image 101. Also, other key indicators determined by the control unit 103 on the basis of impedance measurements, EGR measurements and/or sensor measurements may be displayed.

## Claims

1. Electrical impedance imaging assembly (10) comprising
- at least one electrode patch (11, 21) to be externally applied to a patient, the electrode patch (11, 21) having a plurality of electrodes (12, 22) for measuring impedances of a measurement target placed between the plurality of electrodes (12, 22),
- and a first interface (13) for transmitting electrical signals between the electrode patch (11, 21) and an external device (100) which computes characteristics of the measurement target based on electrical signals of the electrodes (12, 22), in particular a three-dimensional tomographic image (101) of the measurement target,
**characterized in that**
at least a first electrode patch (11) comprises at least 12 electrodes (12), preferably 16-32 electrodes, the electrodes (12) being arranged on a substrate (14) of the first electrode patch (11) in a two-dimensional pattern, preferably in a matrix shape,
and **in that** the first electrode patch (11) comprises an adhesive (15) for fixing the first electrode patch (11) on the skin of the patient, wherein preferably the ratio between the area of the surface (16) of the adhesive for contacting the skin and the area of the total surface (17) of the electrodes (12) facing the skin is 0.5-2.

2. Electrical impedance imaging assembly according to claim 1, wherein the adhesive (15) is arranged on or around each electrode (12) of the first electrode patch.

3. Electrical impedance imaging assembly according to at least one of the previous claims, wherein the electrodes (12, 22) are electrically connectable such that any two electrodes (12, 22) can be used for measuring an impedance there between.

4. Electrical impedance imaging assembly according to at least one of the previous claims, wherein the maximal distance (18) between two electrodes (12) on the first electrode patch (11) which are electrically connectable for measuring an impedance between them, is 10cm to 20cm, preferably 14cm to 16cm;
and/or wherein the minimal distance (19) between two electrodes (12) on the first electrode patch (11) which are electrically connectable for measuring an impedance between them, is 0.3cm to 2.5cm.

5. Electrical impedance imaging assembly according to at least one of the previous claims, wherein
the electrical impedance imaging assembly (10) comprises at least one second electrode patch (21), preferably to be externally applied to the sternum of the patient P, comprising at least one electrode (22) and the second electrode patch (21) being connected to the first electrode patch (11), preferably via a flexible connection (23).

6. Electrical impedance imaging assembly according to claim 5, wherein the at least first electrode patch (11) and the at least second electrode patch (21) are connected by a flexible connection (23), said flexible connection (23) having no electrode, said flexible connection (23) preferably having of a length of at least 200-400mm.

7. Electrical impedance imaging assembly according to claim 5 or 6, wherein
the second electrode patch (21) comprises an adhesive (25) for fixing the electrode patch (21) on the skin of the patient,
preferably arranged on each or around each of the at least one electrode (22),
wherein preferably the ratio between the area of the surface of the adhesive for contacting the skin and the area of the total surface of the electrodes facing the skin is 0.5-2.

8. Electrical impedance imaging assembly according to at least one of the previous claims, wherein at least one electrode patch (11, 22) comprises at least one connection area (30) comprising at least one second interface (31) for removably connecting at least one sensor (32), preferably a temperature, pressure, sound and/or positioning sensor, to be placed at the connection area (30).

9. Electrical impedance imaging assembly according to at least one of the previous claims, wherein at least one sensor (32) is arranged on the electrode patch (11, 21), the sensor (32) preferably being a temperature, pressure, sound or positioning sensor.

10. Electrical impedance imaging assembly according at least one of the previous claims, wherein wires, circuits and/or the electrodes (12, 22) are formed by printing an ink containing a conductive material.

11. An electrical impedance imaging device (200) comprising at least one electrical impedance imaging assembly (10) according to one of claims 1-10 and an external device (100) for receiving an electrical signal measured through the electrical impedance imaging assembly (10), for computing characteristics of the measurement target based on the electrical signals, in particular a three-dimensional tomographic image (101) of the measurement target, wherein the electrical impedance imaging device (200) preferably comprises a display (102) for displaying a tomographic image (101) .

12. An electrical impedance imaging device according to claim 11 comprising a control unit (103) disposed for choosing any two electrodes (12, 22) of the electrical impedance tomography assembly (10) for measuring impedances between the selected electrodes.

13. An electrical impedance imaging device according to one of claims 11-12, comprising a control unit (103) which is designed for receiving an ECG signal from selected electrodes (33) of the electrical impedance tomography assembly (10, 100) .

14. An electrical impedance imaging set comprising at least a first electrical impedance tomography assembly (10) and a second electrical impedance tomography assembly (10) according to one of claims 1-10 and an external device (100) for receiving an electrical signal measured by one of the electrical impedance imaging assemblies (10), for computing characteristics of the measurement target based on the electrical signals, in particular a three-dimensional tomographic image (101) of the measurement target, and preferably external device (100) has a display (102) for displaying a tomographic image (101),
wherein the electrodes of the first electrical impedance tomography assembly (10) are arranged in first pattern and the electrodes of the second electrical impedance tomography assembly (10) are arranged in second pattern, the first pattern being different from the second pattern.

15. Method of manufacturing an electrical impedance imaging assembly, preferably according to one of claims 1-10, comprising the steps of attaching at least 12 electrodes (12), preferably 16-32 electrodes, in a two dimensional pattern, preferably in a matrix shape to the substrate, **characterised in that** an adhesive is attached to or around each electrode, wherein preferably the ratio between the area of the total surface 16 of the adhesive for contacting the skin and the area of the surface (17) of the electrodes (12) facing the skin is 0.5-2.

16. Method of using an electrical impedance imaging assembly (10), preferably according to one of claims 1-10, wherein the electrical impedance imaging assembly (10) comprises at least one electrode patch (11, 21), the electrode patch (11, 21) having at least 12 electrodes (12), preferably 16-32 electrodes, for measuring impedances of a measurement target placed between the plurality of electrodes (12, 22), the electrodes (12) being arranged on a substrate (14) of the first electrode patch (11) in a two dimensional pattern, preferably in a matrix shape, and a first interface (13) for transmitting electrical signals between the electrode patch (11, 21) and an external device (100) which computes characteristics of the measurement target based on electrical signals of the electrodes (12, 22), in particular a three-dimensional tomographic image (101) of the measurement target,
comprising the following steps
(i) placing the electrode patch (11, 21) to the thorax of a patient, such that only a part of the thorax is surrounded by the electrical impedance imaging assembly, in particular less than 180°,
(ii) Fixing the electrode patch (11, 21) to the skin of the patient by an adhesive, the adhesive preferably applied to the first electrode patch (11), such that the ratio between the area of the surface (16) of the adhesive for contacting the skin and the area of the total surface (17) of the electrodes (12) facing the skin is 0.5-2.
